# EUROPEAN PATENT SPECIFICATION

(11) **EP 4 160 206 B1**
(45) Date of publication and mention of the grant of the patent: **03.12.2025**
(21) Application number: 21382875.9
(22) Date of filing: 29.09.2021
(51) Int. Cl.: G01N 33/00, G01N 1/22, G01N 1/02

(54) **PERFUME TESTING DEVICE**
PARFÜMTESTVORRICHTUNG
DISPOSITIF DE TEST DE PARFUM

(43) Date of publication of application: 05.04.2023
(73) Proprietor: Antonio Puig, S.A., 08902 Hospitalet de Llobregat (ES)
(72) Inventor: VIDAL TASA, Jordi, 08030 Barcelona (ES); PANYELLA COSTA, David, 08030 Barcelona (ES); RODENAS SAINZ DE BARANDA, Irene, 08030 Barcelona (ES)
(74) Representative: Bardehle Pagenberg S.L.

(56) References cited:
- EP-A1- 2 249 755
- EP-A1- 3 692 838
- EP-A2- 3 643 275
- KR-A- 20190 031 915
- KR-B1- 101 617 179
- US-A- 5 610 674
- US-B1- 6 325 475
- US-B1- 7 837 649

## Description

### TECHNICAL FIELD

The present disclosure relates to perfume testing devices and more particularly to perfume testing devices configured to hold one or more perfume testers with a perfumed region.

### BACKGROUND

To demonstrate the fragrance notes of a perfume to a user or costumer, perfume contained in vials or spray bottles is generally applied to different types of perfume testers. The perfume testers generally comprise an absorbent material such as an absorbent paper for the user to directly, or indirectly, smell the perfumed applied. A perfume tester such as described in US 6 325 475 B1 can also use digital dispensers to provide different scents to a nasal inhaler to be tested by a user.

The most common type of perfume tester is a strip-like absorbent paper, also known as blotter. Blotters come in a broad variety of shapes, dimensions and materials depending on its use, i.e. weighing strips, soaking strips, or blotters strips for spray. Weighing strips are generally used when same amounts of perfume are to be applied on different instances, including perfumes with different viscosities and densities. Soaking strips may be dipped in a perfume flask until the perfume reaches a certain level generally marked on the surface of the strip. Blotter strips for spray generally come in a wider format than the formers, so that most of the sprayed perfume is deposited on the absorbent material.

During fragrance or perfume demonstrations, a user or consumer usually holds the blotter from the opposite end where the perfume has been applied and waives the blotter to disperse the fragrance through the air. The user can then inhale the surrounding air containing the diffused perfume and perceive the notes or fragrances of the perfume.

One disadvantage of this type of testing system and procedure is that the user's olfactory experience is highly dependent on the surrounding atmosphere and odours contained therein.

In situations wherein perfume demonstrations are performed in a cosmetic boutique, the user is generally surrounded by multiple fragrances of diverse products. Further, during a perfume demonstration the user generally tests more than one perfume in a short time period, leaving a melange of perfumes and fragrances in the surrounding atmosphere that might complicate the correct identification of the notes of each perfume. This effect of perfume mixing can be even more pronounced when a plurality of users are testing perfumes at the same time.

It is also common to perform perfume demonstrations in open public spaces to reach a broader audience. In these situations, the perfume applied to the blotter may quickly disperse into the atmosphere, causing the user to miss subtle fragrances of the perfume or even generate a false sensation of lack of perfume identity. Further, the perfume may even mix with foreign odours, possibly causing an unpleasant experience to the user.

The present disclosure provides devices that at least partially overcome some of the aforementioned drawbacks.

### SUMMARY

The scope of present invention is defined by claims 1-13. A perfume testing device is disclosed. The perfume testing device defines an air inlet, an air outlet and an internal air pathway between the air inlet and the air outlet. Alternatively, the perfume testing device can also be configured to hold a perfume tester with the air inlet. In such a case, the perfume tester is just on the outside of the perfume testing device but next to the air inlet. This configuration allows the air to enter the perfume testing device already impregnated with the perfume. Additionally, the perfume testing device is configured to hold one or more scented perfume testers carrying a fragrance. Furthermore, the air outlet of the perfume testing device is configured to accommodate a nose of a user that during inhalation drives air from the air inlet to the air outlet through the internal air pathway. To this end, the inhalation of the user defines an airflow direction from the air inlet to the air outlet.

Thus, a perfume testing device for inhaling a perfumed airflow is provided. The perfume testing device can improve a user experience by reducing odour disturbances reaching the user. Further, the perfume testing device of the present disclosure takes advantage of perfume convection to increase the perfume intensity in the airflow reaching the user. Furthermore, the perfume testing device reduces the volume of breathing air that has not come into contact with the perfume and potentially allows to use less perfume for the same perfume intensity reaching the nose of the user.

In addition, it is known that during normal breathing i.e., inspiration rate of 200 ml/s or similar, there is a relatively little mixing within the nasal cavity. This leads to significant differences in airflow streamlines inside the nasal cavity depending on the entrance location of the airflow through the nostril. Thus, air directly coming from in front of the nose, rather than from lateral or vertical margins, is generally directed to the olfactory cleft. In this regard, the device of the present disclosure facilitates perfumed air to come from in front of the nose, and therefore enhances the olfactory experience of the consumer.

Additionally, the device of the present disclosure allows to selectively introduce perfume testers with perfumed regions to easily compare fragrances from different perfumes. Besides, the perfume testing device may be used as a tool for perfume development. More precisely, it allows to combine multiple fragrances within the device to test how these fragrances would combine in a new (personalized) perfume.

Thus, users of examples of devices disclosed herein can have an enhanced and more immersive olfactory experience due to a more controlled and conscious inhalation of the perfume.

The one or more scented perfume testers may be arranged in a region of the perfume testing device with a relatively small cross-section area. More specifically, the air gap between the scented perfume testers and the walls of the perfume testing device may be between 2 mm and 5 cm. However, other values for the air gap may also be used depending on the shape and dimensions of the scented perfume testers. Thus, a cross-section of the perfume testing device where the perfume testers are arranged may be between 20 mm² and 10 cm².

In some examples, the length of the region of the perfume testing device configured to receive the scented perfume testers may be between 5 cm and 15 cm, specifically between 7 cm and 12 cm. This length allows that during normal user inhalation, a boundary layer over the scented perfume tester increases its thickness and occupies a substantial portion of the internal air pathway, particularly when combined with the cross-sectional dimensions mentioned before. This enhances fragrance mixing and improves the user olfactory experience. Additionally, in examples in which the air gap between the scented perfume tester and the walls of the perfume testing device is relatively small, i.e. below 2,5 cm or specifically below 2 cm, the boundary layer over the scented perfume tester may mix with a boundary layer developed over a wall of the perfume testing device, generating a turbulent channel flow in which fragrances are distributed throughout the air internal pathway.

In some further examples, the perfume testing device is configured to receive one or more scented perfume testers closely located to a wall, so that most of the incoming air flows over the scented perfume tester, if only one of the surfaces of the testers carries perfume. In further examples, scented perfume testers may be scented on both sides.

In examples, the air outlet of the perfume testing device substantially matches a shape and size of a nose of a user. Thus, a user can fit the nose in the air outlet and, when inhaling, the user may force an air volume to flow around the perfume tester. Then, the perfumed air may flow directly to the user nostrils and nose. The air outlet may fit a nose of a user relatively tight to decrease the amount of breathing air coming from outside the perfume testing device.

Further, the air outlet defines an air outlet cross-section area, the air inlet defines an air inlet cross-section area, and the internal air pathway includes a region of minimum cross-section area. Note that this region of minimum cross-section area does not necessarily represents a minimum cross-section area of the device but of the internal air pathway.

In some examples, the ratio between the air outlet cross-section area and the air inlet cross-section area is between 2 and 15, and more specifically between 5 and 10. This area ratio may not produce a severe increase in pressure drop along the device and may allow the user to breath normally, i.e., without feeling a considerable increase in air resistance while breathing. The difference in cross-section area along the device may increase the air velocity in regions with relatively small cross-section. At the same time, the air through the relatively small cross-section regions may experience a drop in dynamic pressure. Thus, the location of a perfume tester in regions of the device associated with high velocity and low dynamic pressure may lead to a more thorough perfume mixing, wherein perfume molecules of low volatility may still be mixed with the incoming flow.

Further, in other examples, the minimum cross-section area of the internal air pathway may be smaller than the air outlet and air inlet cross-section area. This implies that the airflow at this location, i.e., minimum cross-section area of the air pathway, will tend to accelerate. Thus, when a perfume tester is located in this region, the perfume transfer from the perfume tester to the flow may increase. A ratio between the cross-section area of the air inlet and the minimum cross-section area of the internal air pathway may be between 1 and 10, more specifically between 2 and 5.

In other examples, the perfume testing device comprises a support structure to hold the perfume tester. The support structure may not contact the perfumed region. This allows replacing the perfume tester easily, without the need of cleaning the perfume testing device between subsequent smells. This may facilitate a quick comparison between perfumes in the customer's benefit.

In some examples, the internal air pathway is defined by walls comprising at least one slot configured to receive a perfume tester. The walls may be made of a substantially impermeable material such as polymers, cellulose base materials with surface treatments, ceramic materials, metal materials and others. Herein, when the walls comprise a plurality of slots, the slots may be distributed along the airflow direction. This configuration allows placement of several perfume testers at different distances from the air outlet, so that the respective fragrances on them may reach the nose of the user with different intensities due to perfume intensity decay.

The perfume testing device also comprises one or more perfume testers with a perfumed region. The perfume tester may be made of a perforated material, as for example perforated paper. A perforated material may have a larger wetted area than a non-perforated material, leading to an enhanced perfume mixing and transport through the airflow. Further, the perforated material may be located substantially perpendicular to the flow direction without fully blocking the air pathway. Additionally, a perforated material may also perturb the flow around it, acting as a turbulence inducer and further enhancing perfume mixing.

In accordance with the present invention, the one or more perfume testers comprise strips of absorbent paper that is able to absorb and release a scent, e.g. a liquid scent. In some examples, a perfume tester may be a blotting paper, e.g. comprising cellulose. Outside the scope of the present invention, a perfume tester may include porous or perforated materials such as ceramic, porous plastic, wood, fabric and even concrete. If solid scents are used, a perfume tester may not need to absorb and release the smell of the solid scents. The perfume tester may also comprise a region free from perfume to handle or hold the perfume tester without perfume cross-contamination. Said region free from perfume may be a non-absorbent region or layer.

Throughout this disclosure, a scent or fragrance may mean a substance, e.g. a liquid or solid, with a certain smell that is deposited on a blotter and combined with other different scents in order to create a (personalized) perfume. A perfume may therefore be understood as a substance, e.g. a liquid or solid, which results from combining two or more scents or fragrances, and in particular the smells of the two or more scents or fragrances. Liquid perfumes may be either alcohol based, water based, or oil based. Solid scents or solid perfumes may include a solid substance able to absorb and release a smell. In an example, a solid scent or perfume is scented wax. Also, a new perfume may be obtained by mixing e.g. two existing commercial perfumes. In such a case, each of the two already existing perfumes may be considered a scent. The terms "scent" and "fragrance" are also used herein to cover complete perfumes (e.g. commercially available perfumes), perfumery ingredients (e.g. bergamot, citron, neroli, sandalwood,...), basic combinations of perfumery ingredients, which may also be known as "notes" (e.g. citrus, floral, musk,...) and more complex combinations of perfumery ingredients or combinations of notes, which may be also known as "accords" (e.g. oriental, amber, ...).

### BRIEF DESCRIPTION OF THE DRAWINGS

Non-limiting examples of the present disclosure will be described in the following, with reference to the appended figures, in which:
Figure 1 schematically illustrates a perspective anterior view of an example of a perfume testing device according to the present disclosure;
Figures 2 schematically illustrates a perspective anterior view of a further example of a perfume testing device according to the present disclosure;
Figures 3 schematically illustrates a perspective posterior view of another example of a perfume testing device according to the present disclosure;
Figure 4 schematically illustrates a perspective posterior view of yet a further example of a perfume testing device according to the present disclosure;
Figure 5 schematically illustrates a perspective anterior view of an additional example of a perfume testing device according to the present disclosure; and
Figures 6 schematically illustrates a perspective posterior view of another additional example of a perfume testing device according to the present disclosure.

### DETAILED DESCRIPTION OF EXAMPLES

Reference now will be made in detail to embodiments of the disclosure, one or more examples of which are illustrated in the drawings. Each example is provided by way of explanation, not as a limitation. In fact, it will be apparent to those skilled in the art that various modifications and variations can be made in the present disclosure without departing from the scope of the appended claims. For instance, features illustrated or described as part of one embodiment can be used with another embodiment to yield a still further embodiment. Thus, it is intended that the present disclosure covers such modifications and variations as come within the scope of the appended claims. In general, the same reference signs have been used to denote the same or similar elements across the figures.

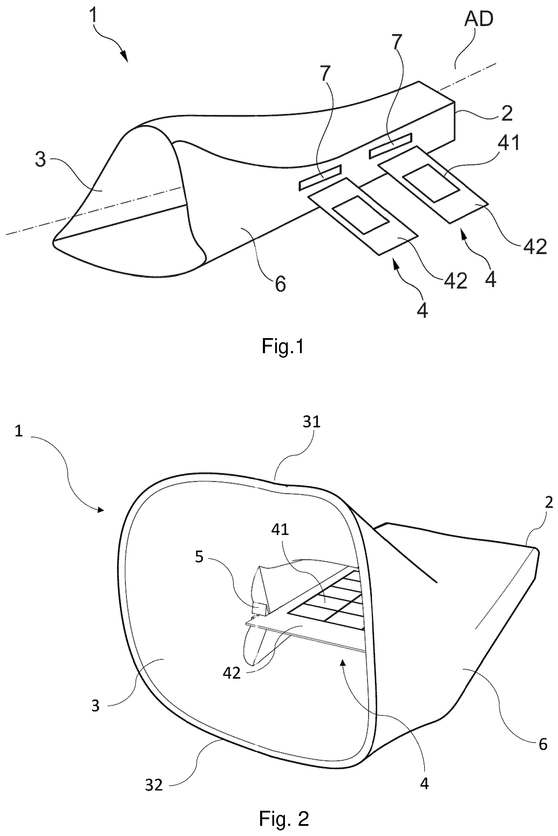

Figure 1 schematically illustrates a perfume testing device 1 according to the present disclosure. It may be seen that the perfume testing device 1 comprises an air inlet 2, an air outlet 3 and an internal air pathway between the air inlet 2 and the air outlet 3. The air outlet 3 is designed so as to fit relatively tight a nose of a user, but air outlets with different geometries that may be suitable to simply accommodate a nose of a user can also be used. Further, the air outlet 3 of the perfume testing device may be configured to accommodate a mouth and a nose of a user. Thus, when a user breathes and inhales air, the user drives an airflow from the air inlet 2 to the air outlet 3. As may be seen in figure 1, this airflow defines an airflow direction AD. The airflow direction AD may be substantially straight, as in the example shown in figure 1. However, the airflow direction AD may follow a more complex internal path, depending on specific configurations of the perfume testing device 1 and its internal geometry.

In the example shown in figure 1, the perfume testing device 1 comprises substantially impermeable walls 6. The walls 6 may be made of a broad range of materials including polymers, cellulose base materials, metals, ceramics or other. The rigidity of the material may be such as to provide structural rigidity during use and avoid the collapse of the perfume testing device 1 during the perfume inhalation.

Figure 1 shows that the perfume testing device 1 is configured to hold one or more perfume testers 4 between the air inlet 2 and the air outlet 3. However, the perfume testing device 1 can also be configured to hold a perfume tester 4 with the air inlet 2, as will be explained in view of figure 2. In such a case, the perfume tester 4 may be just on the outside of the perfume testing device 1 but next to the air inlet 2. This arrangement allows the air to enter the perfume testing device 1 already impregnated with the perfume.

Besides, figure 1 shows that the perfume testing device 1 may comprise one or more slots 7 to receive a perfume tester 4. For illustration purposes, the perfume testers 4 are shown horizontally displaced from its location during use. In the present example, the perfume testers 4 are made of an absorbent layer 41 with a perfumed region and a portion 42 free from perfume. The portion 42 may be used as a contact region with the perfume testing device 1 to avoid perfume and device 1 cross-contamination. Outside the scope of the present invention, the portion 42 of the perfume tester 4 may comprise a non-absorbent layer. Besides, the perfume may be applied in a central region of the perfume tester 4 to limit the spread of the perfume on the absorbent layer 41. Thus, the perimeter of the perfume tester 4 may remain free from perfume, even in absence of non-absorbent layer.

In this example, the slots 7 are located in a lateral wall 6 and are distributed along the airflow direction AD. This distribution of slots 7 causes the perfume in the absorbent layer 41 located further upstream (closer to the air inlet 2) to mix with the airflow earlier than the perfume in the perfume tester 4 downstream. This difference in distance from the airflow-perfume mixing point to the nose of the user can be used to identify subtle changes in the perfume notes with perfume intensity decay. Alternatively, the slots 7 can be arranged perpendicular to the airflow direction AD, so that all perfumes are substantially located at the same distance from the air outlet 3, and therefore from the user. This configuration allows the user to experience a mixture of perfumes with individual perfumes having a similar intensity relative to each other. The slots 7 may be arranged on both lateral walls 6 so that the perfume tester 4 can go through the perfume testing device 1 perpendicular to the airflow direction AD. Doing so, the ends of the perfume tester 4 may be at least partially supported by the slots 7 themselves, without the need of additional support structures.

In this example, the slots 7 may be in a region corresponding to the minimum cross-section area of the internal air pathway. As previously discussed, if the minimum cross-section area is smaller than the air inlet cross-section area, the airflow coming from the air inlet 3 may tend to accelerate in this region enhancing perfume transfer from the perfume testers 4 to the airflow. To reduce clutter, the internal walls defining the internal air pathway are not illustrated in figure 1. The internal walls may define a curved air pathway or an air pathway comprising walls at an angle. Other configurations wherein the internal pathway does not comprise a minimum cross-section area as previously disclosed can also be used.

Additionally, at least one of the slots 7 may be configured to be selectively open and closed. Thus, the slots 7 may allow to selectively create paths of communication between the inside and the outside of the perfume testing device 1. In this way, a slot 7 may remain closed when it does not include a perfume tester 4. Therefore, the internal air pathway between the air inlet 2 and the air outlet 3 of the perfume testing device 1 is maintained. Further, any slot 7 may also be configured to substantially match the geometry of a perfume tester 4, so that the slot 7 is at least partially closed when the perfume tester 4 is introduced into it. Furthermore, the slots 7 can also be configured to be closed after the introduction of the perfume tester 4.

Alternative configurations in which the perfume testing device 1 comprise slots 7 arranged in other locations are also possible. Further, the slots 7 may have any suitable shape, i.e. triangular, rectangular, pentagonal, hexagonal, circular and other non-regular geometry.

Alternatively, other configurations in which the perfume testing device 1 does not comprise slots 7 are also disclosed. For example, the perfume tester 4, i.e., a strip-like blotter, a rectangular blotter or other, may be introduced directly from the air inlet 2 or air outlet 3. The perfume testing device 1 may comprise support structures 5, guides or other elements to hold in place the perfume tester 4.

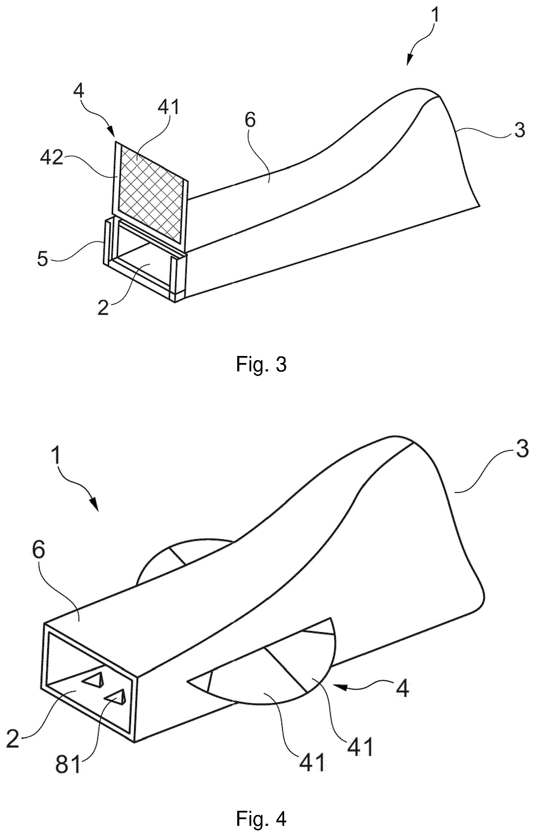

The support structures 5 may be internal and/or external. An internal structure may be configured to hold the perfume tester 4 substantially inside the perfume testing device 1, whereas an external structure may be configured to hold the perfume tester 4 substantially outside the perfume testing device 1, so that the airflow enters the air inlet 2 already containing perfume. Similarly, in other configurations the perfume tester 4 can be held in the outside of the perfume testing device 1, as will be discussed for figure 2 and 4.

In the example of figure 1, and in further examples, the perfume testing device forms a substantially tubular body, i.e. an elongated hollow body delimited by one or more sidewalls. The tubular body has a hollow cross-section which may have any suitable shape, e.g. rounded, square, rectangular, and other.

At or near a distal end of the tubular body, the air inlet may be arranged. A perfume tester such as a blotter may be arranged in a portion of the tubular body that is configured to increase or maximize perfume transfer. This portion of the tubular body may be denominated the mixing chamber or mixing zone. The cross-sectional area of the mixing zone may be between 20mm² and 10 cm². More specifically, the air gap between the scented perfume testers and the walls of the perfume testing device in the mixing zone may be between 2 mm and 5 cm.

In examples, the tubular body may have a substantially constant cross-sectional area (and in examples also a substantially constant cross-section) from the location of the air inlet to the mixing zone. Downstream from the mixing zone (closer to the proximal end of the tubular body), the cross-sectional area may increase towards the outlet. The outlet as mentioned before may fit a nose of a user.

The outlet may be parallel to an air inlet. The outlet does not need to be perpendicular to the air flow direction through the tubular body. The outlet may be adapted to follow a face line of consumers.

Figure 2 schematically illustrates an anterior perspective view of another example of a perfume testing device 1 according to the present invention. The exemplary testing device 1 comprises an air inlet 2 with rectangular cross-section. Further, the device 1 may comprise an internal support structure 5 configured to receive a perfume tester 4. In this example, the internal support structure 5 includes guides located along the air pathway. The guides may extend from the air inlet 2, so that a perfume tester can be introduced through the same. In other examples, the guides may be configured to receive a perfume tester from the air outlet 3. The guides in the present example are configured to contact a portion 42 of the perfume tester 4 which is not impregnated with perfume. Doing so, the guides may remain free from fragrances and the perfume testing device 1 may be used for different fragrances without cleaning it and avoiding fragrance contamination. Further, in the present example, the guides are substantially in a central position relative to the top and bottom walls. However, the guides may be arranged closer to one of the top and bottom walls, so that the absorbent layer 41 with perfume may face the largest air gap.

Further, figure 2 shows that the perfume testing device 1 comprises an air outlet 3 with a periphery that substantially mates with a nose of a user. The air outlet 3, in particular, an upper edge of the air outlet 3 may include an upper recess 31 to mate a nasal ridge or a nasal bridge of a user. Additionally, the periphery of the air outlet 3, in particular the lower edge of the air outlet 3, may also include a curved edge 32 to mate with a philtrum of a user.

Alternatively, the periphery of the air outlet 3 may include a curved edge to contact under an inferior lip of the user. More precisely, the periphery of the air outlet 3 may mate with a labiomental groove of a user.

In some examples, the periphery of the air outlet may additionally or alternatively include a flexible layer to promote a better fit between the air outlet 3 and the nose or mouth of a user. More specifically, the flexible layer may include a layer of natural or synthetic sponge, or elastomer materials such as silicone.

The example illustrated in figure 2 has a cross-section area ratio between the air outlet 3 and air inlet 2 of approximately 5, but other area ratios may also be used.

Figure 3 schematically illustrates a posterior perspective view of another example of a perfume testing device 1 according to the present invention. The perfume testing device 1 comprises a rectangular cross-section air inlet 2, but air inlets with other cross-section geometries can also be used.

In the example shown in figure 3, the perfume testing device 1 also comprises a perfume tester 4 and a support structure 5 located in the vicinity of the air inlet 2. For illustration purposes, the perfume tester 4 is shown vertically displaced from its location during use. The perfume tester 4 comprises an absorbent layer 41 with a perfumed region and a portion 42 free from perfume partially surrounding the absorbent layer 41. The support structure 5 is configured to hold the perfume tester 4 without making contact with the absorbent layer 41. In other instances, the portion 42 free from perfume may fully surround the absorbent layer 41.

In this example, the absorbent layer 41 is a mesh, or perforated screen, that allows air from the outside to go through the perfume tester 4 and inside the perfume testing device 1. As discussed in view of figure 1, the portion 42 may remain free from perfumes and can be used to contact the support structure 5 of the perfume testing device 1. This allows to use multiple perfume testers 4 sequentially without cleaning the perfume testing device 1 and without cross-contamination.

Figure 4 schematically illustrates a posterior perspective view of yet another example of the perfume testing device 1 according to the present disclosure. An alternative perfume testing device 1 comprises a perfume tester 4 in the shape of a rotatable wheel. In the present disclosure, the term rotatable wheel is understood as a rotatable element substantially contained in a flat (2D) plane. The rotatable wheel has a substantially circular periphery, an elliptic periphery, a periphery with multiple lobes or other regular or irregular geometries.

The perfume tester 4 is arranged with the air outlet 3, and in this alternative embodiment, is partially located upstream the air outlet 3. The central region of the wheel is located inside the perfume testing device 1, whereas certain peripheric areas of the rotatable wheel remain outside the perfume testing device 1. Further, the rotatable wheel is divided in circular sectors, where each circular sector comprises an absorbent layer 41 with a perfumed region. The circular sectors can be separated between them so that the perfume of each circular sector does not mix with the perfume of the vicinity sectors. To avoid the cross-contamination of circular sectors, each circular sector may be at least partially surrounded by a non-absorbent material.

Thus, the rotatable wheel may be selectively rotated to include a given absorbent layer 41 with a perfumed region within the internal air pathway. Thus, the rotation of the perfume tester 4 can place a different perfume in front of the air inlet 2 for the user to quickly compare fragrances.

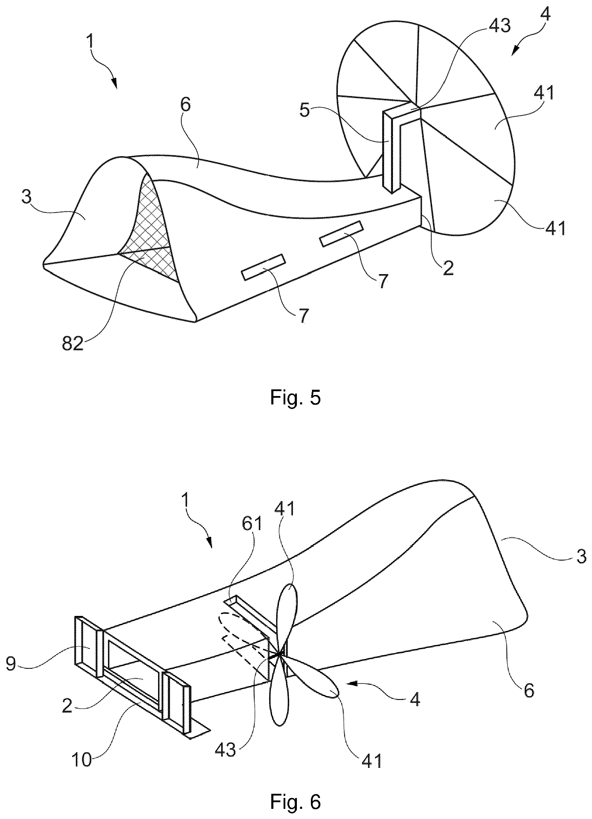

Figure 4 also illustrates that the perfume testing device may comprise turbulence inducers. In the present example, the turbulence inducers are located in the internal air pathway downstream the air inlet 2. In this example, the turbulence inducers are vortex generators 81 with indentations protruding from a wall 6 towards the internal air pathway disturbing the flow. In the present example, the turbulence inducers 81 have a pyramidal geometry but rectangular blocks, conical sections and other geometries could also be used. The turbulence inducers 81 may be located on any of the walls 6 and may be arranged upstream or downstream of the perfume tester 4. Other types of turbulence inducers such as serrated profiles of different dimensions or perforated screens can also be used, as will be seen explained in view of figure 5. Similarly, sudden variations of the cross section of the air internal pathway such as backward or forward-facing steps can also be used to disturb the airflow and enhance perfume mixing.

Figure 5 schematically illustrates an anterior perspective view of another example of a perfume testing device 1 according to the present invention. The perfume testing device 1 shown comprises a support structure 5 to hold a perfume tester 4 in a shape of a rotatable wheel outside the perfume testing device 1. The perfume tester 4 comprises circular sections which, in turn, comprise absorbent layers 41. The rotation of the perfume tester 4 facilitates the positioning of different perfumed regions (corresponding to discrete absorbent layers 41) in front of the air inlet 2. Thus, a user can easily change the perfume to smell by rotating the perfume tester 4. To enhance perfume mixing and to ease the penetration of external air inside the perfume testing device 1, the absorbent layers 41 in this example are perforated. The degree of perforation, also known as open area, may be chosen within a range that does not severely increase the pressure drop along the airflow direction AD. Thus, open areas above 40% may be selected, specifically above 50% and even more specifically above 60%.

Additionally, the perfume testing device 1 of the present example also comprises slots 7 in the walls 6, where further perfume testers (not illustrated) may be located. The provision of more than one perfume tester allows testing a melange of perfumes. As an example, a user may introduce a perfume tester in a slot 7 as a base perfume. Then, the user may choose a plurality of additional perfumes to be applied on the rotatable wheel 4. Thus, by rotating of the rotatable wheel 4 the user may be able to compare several perfumes or combination of perfumes over the base perfume. The number of base perfumes can change depending on the number of slots 7 and the perfume testers 4 introduced on them.

In the example of figure 5, the perfume testing device 1 also comprise a perforated screen 82. The perforated screen 82 is located downstream the slots 7, but the perforated screen 82 may be alternatively or additionally located upstream of the same, as the turbulence inducers 81 illustrated in figure 4. Perforated screens 82 and meshes with different open area can be used depending to the mixing requirements. Mesh open areas may be from 10% to 90%, specifically from 30% to 80% and even more specifically from 40 to 70%. The perforation of the screens might comprise aligned or staggers perforations. Further, the geometry of the perforation might be round, square, hexagonal, slot shape or others. The mesh or perforated screen 82 may be made of a textile-based material, a cellulose-based material, a polymer-based material, a metal-based material or others known in the art. The combination of different kinds of turbulence inducers, i.e. vortex generators 81, perforated screens 82, backward or forward-facing steps, can also be used.

Figure 6 is an anterior perspective view of yet a further example of a perfume testing device 1 according to the present invention. The perfume testing device 1 comprises a perfume tester 4 in a shape of a rotatable lobular wheel. The rotatable lobular wheel rotates about an axis of rotation 43 that may be located on a wall 6. Alternatively, the perfume testing device 1 may also comprise a support structure, as shown in figure 5, to hold the rotatable lobular wheel in place.

In this example, the walls comprise a cut 61 providing enough clearance for the rotatable lobular wheel to go through. The length and width of the cut 61 may be determined by the size of the rotatable lobular wheel and the structural properties of the wall 6.

In figure 6, the rotatable lobular wheel comprises four lobes with a radial gap between each other, but any other number, shape and arrangement of lobes can also be used. The radial gap between lobes may allow to position a single lobe inside the perfume testing device 1. Further, each lobe may comprise an absorbent layer 41 with a distinct perfumed applied on it. The configuration of the rotatable lobular wheel shown in the example reduces significantly the cross-contamination between perfumes from different lobes.

Additionally, the example in figure 6 shows that the air inlet 2 of the perfume testing device 1 may comprise elements for increasing or decreasing the air inlet cross-section area. In this example, this is achieved by means of slidable elements 9 located on a guide 10. Other elements for modifying the cross-section area of the air inlet 2 can also be used. These elements may provide discrete variations in the cross-section area or a continuous variation in the cross-section area. In other examples, discrete variations in the cross-section area can be provided by a rotatable plate comprising several openings radially distributed. Thus, the rotation of the plate may enable to selectively align one of said openings with the air inlet 2. Therefore, the effective area of the air inlet 2 might be modified. Further, each of said openings may have a different cross-section area. In another example, a continuous variation in the cross-section area of the air inlet 2 might be provided by means of a multi blade diaphragm coupled to the air inlet 2.

Other non-rotating mechanisms to modify the cross-section area of the air inlet such as a linear moving mask or knife-edge can also be used. Diaphragms and other means to define an air inlet 2 with variable cross-section might be implemented in the air inlet 2 irrespectively of the cross-section geometry of the same, i.e. rectangular, circular or other. The variation of the open area of the air inlet 2 may change the pressure drop across the device and may modify the velocity of the air across the air inlet 2. Thus, under certain circumstances, an open area of variable cross-section can lead to a more pronounced mixing due to the generation of an incoming turbulent flow.

The scope of the present disclosure should not be limited by particular examples, but should be determined only by a fair reading of the claims that follow.

## Claims

1. A perfume testing device (1) forming a substantially tubular body delimited by one or more sidewalls and comprising an air inlet (2), an air outlet (3) and an internal air pathway between the air inlet (2) and the air outlet (3), wherein
the perfume testing device (1) is configured to hold one or more scented perfume testers (4) between the air inlet (2) and the air outlet (3) or with the air inlet (2), and
the air outlet (3) is configured to accommodate a nose of a user inside, the nose of the user during inhalation driving air from the air inlet (2) to the air outlet (3) through the internal air pathway defining an airflow direction (AD),
**characterised in that** the one or more scented perfume testers (4) comprise one or more strips of absorbent paper (41) configured to absorb and release a scent or a rotatable wheel at least partially located upstream the air outlet (3), wherein the rotatable wheel is divided in circular sectors or lobes, each circular sector or lobe comprising an absorbent layer (41) with a perfumed region.

2. The perfume testing device (1) according to claim 1, wherein the air outlet (3) defines an air outlet cross-section area, the air inlet (3) defines an air inlet cross-section area, and the internal air pathway includes a region of minimum cross-section area, and wherein a ratio between the air outlet cross-section area and the air inlet cross-section area is between 1 and 15, specifically between 5 and 10.

3. The perfume testing device (1) according to any of claims 1 and 2, wherein the air outlet (2) substantially matches a shape and a size of a nose of a user.

4. The perfume testing device (1) according to any of claims 1 - 3, wherein the device comprises a support structure (5) to hold the perfume tester (4) without the support structure (5) contacting with a perfumed region of the perfume tester (4).

5. The perfume testing device (1) according to any of claims 1 - 4, wherein the perfume testing device (1) is configured to hold one or more perfume testers (4) at a distance of between 2 mm and 5 cm from internal walls delimiting the internal air pathway.

6. The perfume testing device (1) according to any of claims 1 - 5, wherein the minimum cross-section area of the internal air pathway is smaller than the air outlet and air inlet cross-section area.

7. The perfume testing device (1) according to any of claims 1 - 6, wherein the internal air pathway is defined by walls (6) comprising at least one slot (7) configured to receive a perfume tester (4).

8. The perfume testing device (1) according to claim 7, wherein two or more of the slots (7) are distributed along the airflow direction (AD) or perpendicular to the airflow direction (AD).

9. The perfume testing device (1) according to any of claims 7 and 8, wherein at 5 least one slot (7) is configured to be selectively open and closed.

10. The perfume testing device (1) according to any of claims 1 - 12, further comprising turbulence inducers (81) located in the internal air pathway upstream the air outlet (3).

## Patentansprüche

1. Parfümtestvorrichtung (1), die einen im Wesentlichen röhrenförmigen Körper bildet, der durch eine oder mehrere Seitenwände begrenzt ist und einen Lufteinlass (2), einen Luftauslass (3) und einen inneren Luftweg zwischen dem Lufteinlass (2) und dem Luftauslass (3) umfasst, wobei
die Parfümtestvorrichtung (1) dazu ausgelegt ist, einen oder mehrere duftende Parfümtester (4) zwischen dem Lufteinlass (2) und dem Luftauslass (3) oder mit dem Lufteinlass (2) zu halten, und
der Luftauslass (3) dazu ausgelegt ist, die Nase eines Benutzers im Inneren aufzunehmen, wobei die Nase des Benutzers beim Einatmen Luft von dem Lufteinlass (2) zu dem Luftauslass (3) durch den inneren Luftweg treibt, der eine Luftströmungsrichtung (AD) definiert, **dadurch gekennzeichnet, dass** der eine oder die mehreren duftenden Parfümtester (4) einen oder mehrere Streifen aus saugfähigem Papier (41), die dazu ausgelegt sind, einen Duft zu absorbieren und freizusetzen, oder ein drehbares Rad umfassen, das sich zumindest teilweise stromaufwärts des Luftauslasses (3) befindet, wobei das drehbare Rad in kreisförmige Sektoren oder Flügel unterteilt ist und jeder kreisförmige Sektor oder Flügel eine saugfähige Schicht (41) mit einem parfümierten Bereich umfasst.

2. Parfümtestvorrichtung (1) nach Anspruch 1, wobei der Luftauslass (3) eine Luftauslassquerschnittsfläche definiert, der Lufteinlass (3) eine Lufteinlassquerschnittsfläche definiert und der innere Luftweg einen Bereich mit minimaler Querschnittsfläche umfasst, und wobei ein Verhältnis zwischen der Luftauslassquerschnittsfläche und der Lufteinlassquerschnittsfläche zwischen 1 und 15, insbesondere zwischen 5 und 10, liegt.

3. Parfümtestvorrichtung (1) nach einem der Ansprüche 1 und 2, wobei der Luftauslass (2) im Wesentlichen einer Form und Größe einer Nase eines Benutzers entspricht.

4. Parfümtestvorrichtung (1) nach einem der Ansprüche 1-3, wobei die Vorrichtung eine Trägerstruktur (5) zum Halten des Parfümtesters (4) umfasst, ohne dass die Trägerstruktur (5) mit einem parfümierten Bereich des Parfümtesters (4) in Kontakt kommt.

5. Parfümtestvorrichtung (1) nach einem der Ansprüche 1-4, wobei die Parfümtestvorrichtung (1) dazu ausgelegt ist, einen oder mehrere Parfümtester (4) in einem Abstand zwischen 2 mm und 5 cm von den Innenwänden zu halten, die den inneren Luftweg begrenzen.

6. Parfümtestvorrichtung (1) nach einem der Ansprüche 1-5, wobei die minimale Querschnittsfläche des inneren Luftwegs kleiner ist als die Luftauslass- und Lufteinlassquerschnittsfläche.

7. Parfümtestvorrichtung (1) nach einem der Ansprüche 1-6, wobei der innere Luftweg durch Wände (6) definiert ist, die mindestens einen Schlitz (7) aufweisen, der zum Aufnehmen eines Parfümtesters (4) ausgelegt ist.

8. Parfümtestvorrichtung (1) nach Anspruch 7, wobei zwei oder mehr der Schlitze (7) entlang der Luftströmungsrichtung (AD) oder senkrecht zu der Luftströmungsrichtung (AD) verteilt sind.

9. Parfümtestvorrichtung (1) nach einem der Ansprüche 7 und 8, wobei mindestens ein Schlitz (7) dazu ausgelegt ist, wahlweise geöffnet und geschlossen zu sein.

10. Parfümtestvorrichtung (1) nach einem der Ansprüche 1-12, die ferner Turbulenzerzeuger (81) umfasst, die sich im inneren Luftweg stromaufwärts des Luftauslasses (3) befinden.

## Revendications

1. Dispositif de test de parfum (1) formant un corps sensiblement tubulaire délimité par une ou plusieurs parois latérales et comprenant une entrée d'air (2), une sortie d'air (3) et un passage d'air interne entre l'entrée d'air (2) et la sortie d'air (3), dans lequel
le dispositif de test de parfum (1) est conçu pour maintenir un ou plusieurs testeurs de parfum parfumés (4) entre l'entrée d'air (2) et la sortie d'air (3) ou avec l'entrée d'air (2), et
la sortie d'air (3) est conçue pour recevoir le nez d'un utilisateur à l'intérieur, le nez de l'utilisateur entraînant, pendant l'inhalation, l'air de l'entrée d'air (2) vers la sortie d'air (3) à travers le passage d'air interne définissant une direction d'écoulement d'air (AD), **caractérisé en ce que** le ou les testeurs de parfum parfumés (4) comprennent une ou plusieurs bandelettes de papier absorbant (41) conçues pour absorber et libérer un parfum ou une roue rotative située au moins partiellement en amont de la sortie d'air (3), la roue rotative étant divisée en secteurs ou lobes circulaires, chaque secteur ou lobe circulaire comprenant une couche absorbante (41) comportant une région parfumée.

2. Dispositif de test de parfum (1) selon la revendication 1, dans lequel la sortie d'air (3) définit une zone de section transversale de sortie d'air, l'entrée d'air (3) définit une zone de section transversale d'entrée d'air et le passage d'air interne comprend une région de zone de section transversale minimale, et dans lequel un rapport entre la zone de section transversale de sortie d'air et la zone de section transversale d'entrée d'air est compris entre 1 et 15, plus précisément entre 5 et 10.

3. Dispositif de test de parfum (1) selon l'une quelconque des revendications 1 et 2, dans lequel la sortie d'air (2) correspond sensiblement à la forme et à la taille du nez d'un utilisateur.

4. Dispositif de test de parfum (1) selon l'une quelconque des revendications 1 à 3, le dispositif comprenant une structure de support (5) pour maintenir le testeur de parfum (4) sans que la structure de support (5) n'entre en contact avec une région parfumée du testeur de parfum (4).

5. Dispositif de test de parfum (1) selon l'une quelconque des revendications 1 à 4, dans lequel le dispositif de test de parfum (1) est conçu pour maintenir un ou plusieurs testeurs de parfum (4) à une distance comprise entre 2 mm et 5 cm des parois internes délimitant le passage d'air interne.

6. Dispositif de test de parfum (1) selon l'une quelconque des revendications 1 à 5, dans lequel la zone de section transversale minimale du passage d'air interne est plus petite que la zone de section transversale de sortie d'air et d'entrée d'air.

7. Dispositif de test de parfum (1) selon l'une quelconque des revendications 1 à 6, dans lequel le passage d'air interne est défini par des parois (6) comprenant au moins une fente (7) conçue pour recevoir un testeur de parfum (4).

8. Dispositif de test de parfum (1) selon la revendication 7, dans lequel deux des fentes (7) ou plus sont réparties le long de la direction du flux d'air (AD) ou perpendiculairement à la direction du flux d'air (AD).

9. Dispositif de test de parfum (1) selon l'une quelconque des revendications 7 et 8, dans lequel au moins une fente (7) est conçue pour être sélectivement ouverte et fermée.

10. Dispositif de test de parfum (1) selon l'une quelconque des revendications 1 à 12, comprenant en outre des inducteurs de turbulence (81) situés dans le trajet d'air interne en amont de la sortie d'air (3).
